# EUROPÄISCHE PATENTSCHRIFT

(11) **EP 3 030 191 B1**
(45) Veröffentlichungstag und Bekanntmachung des Hinweises auf die Patenterteilung: **09.08.2017**
(21) Anmeldenummer: 14737141.3
(22) Anmeldetag: 20.06.2014
(51) Int. Cl.: A61B 90/30

(54) **VERFAHREN FÜR EINE KALIBRIERUNG EINER SENSORVORRICHTUNG FÜR DIE REGELUNG DER AUSRICHTUNG EINER AUSLEUCHTVORRICHTUNG UND AUSLEUCHTSYSTEM**
METHOD FOR CALIBRATING A SENSOR DEVICE FOR REGULATING THE ALIGNMENT OF AN ILLUMINATION DEVICE AND ILLUMINATION SYSTEM
PROCÉDÉ D'ÉTALONNAGE D'UN SYSTÈME DE CAPTEUR SERVANT À RÉGLER L'ORIENTATION D'UN DISPOSITIF D'ÉCLAIRAGE ET SYSTÈME D'ÉCLAIRAGE

(30) Priorität: 22.06.2013 DE 102013010515
(43) Veröffentlichungstag der Anmeldung: 15.06.2016
(73) Patentinhaber: Drägerwerk AG & Co. KGaA, 23558 Lübeck (DE)
(72) Erfinder: SCHLICHTING, Stefan, 23566 Lübeck (DE); SCHLAEFER, Alexander, 23617 Stockelsdorf (DE); HARTMANN, Florian, 23558 Lübeck (DE)
(74) Vertreter: Mildner, Volker
(86) Internationale Anmeldenummer: PCT/EP2014/001687
(87) Internationale Veröffentlichungsnummer: WO 2014/202228

(56) Entgegenhaltungen:
- EP-A1- 2 283 790
- BENNET TILLAPAUGH ET AL: "Indirect camera calibration for surgery tracking", BIOMEDICAL IMAGING: FROM NANO TO MACRO, 2009. ISBI '09. IEEE INTERNATIONAL SYMPOSIUM ON, IEEE, PISCATAWAY, NJ, USA, 28. Juni 2009 (2009-06-28), Seiten 1083-1086, XP031502236, ISBN: 978-1-4244-3931-7
- JWU-SHENG HU ET AL: "Kinematic calibration of manipulator using single laser pointer", INTELLIGENT ROBOTS AND SYSTEMS (IROS), 2012 IEEE/RSJ INTERNATIONAL CONFERENCE ON, IEEE, 7. Oktober 2012 (2012-10-07), Seiten 426-430, XP032287377, DOI: 10.1109/IROS.2012.6385531 ISBN: 978-1-4673-1737-5

## Beschreibung

Die vorliegende Erfindung betrifft ein Verfahren für die Kalibrierung einer Sensorvorrichtung für die Regelung der Ausrichtung einer Ausleuchtvorrichtung zur Veränderung der Position eines durch die Ausleuchtvorrichtung in einer Ausleuchtrichtung erzeugten Ausleuchtbereichs sowie ein entsprechendes Ausleuchtsystem mit einer solchen Ausleuchtvorrichtung, einer Sensorvorrichtung und einer entsprechenden Kontrolleinheit.

Es ist grundsätzlich bekannt, dass z. B. in einem Operationssaal automatisiert verstellbare Ausleuchtsysteme vorgesehen werden. Diese weisen eine Ausleuchtvorrichtung in Form einer Operationslampe auf. Mithilfe dieser Operationslampe als Ausleuchtvorrichtung kann in Ausleuchtrichtung ein Ausleuchtbereich erzeugt werden. Für den Einsatz einer solchen Ausleuchtvorrichtung muss der Ausleuchtbereich an einen definierten Ort verschoben werden. Dies geschieht bei bekannten Ausleuchtvorrichtungen durch eine manuelle Verstellung der Ausleuchtrichtung, um den Ausleuchtbereich mit einem gewünschten auszuleuchtenden Bereich zur Deckung zu bringen.

Nachteilhaft bei bekannten Ausleuchtvorrichtungen ist es, dass die manuelle Einflussnahme auf die Ausleuchtvorrichtung einen Griff des Bedienpersonals an die Ausleuchtvorrichtung erfordert. Wird die Ausleuchtvorrichtung z. B. in einem sterilen Raum, z. B. in einem OP-Bereich, eingesetzt, so bedeutet dieser Handgriff eine Bewegung aus dem Sterilbereich heraus.

Dementsprechend ist bereits vorgeschlagen worden, dass über Gestensteuerungen eine automatisierte Verstellung des Ausleuchtbereichs erfolgen kann. Jedoch erfordert eine automatisierte Verstellung eine Aktuierung hinsichtlich der Verstellung der Ausrichtung der Ausleuchtvorrichtung. Hierfür ist z. B. ein Roboterarm vorgesehen, welcher in seinem Koordinatensystem Stellbewegungen zur Veränderung der Ausleuchtrichtung der Ausleuchtvorrichtung durchführt. Gleichzeitig muss eine Kontrolle bzw. eine Vorgabe einer Sensorvorrichtung erfolgen, um die reale Veränderung der Ausleuchtrichtung und damit eine Verschiebung des Ausleuchtbereichs zu erzielen, zu erzeugen bzw. zu überwachten. Hierfür sind bekannterweise Sensorvorrichtungen, z. B. in Form von Kamerasystemen, im Einsatz.

Nachteilhaft bei den bereits bekannten automatisierten Veränderungsverfahren für die Ausleuchtvorrichtungen ist die aufwendige Kalibrierung. So muss zwischen dem Koordinatensystem der Sensorvorrichtung einerseits und dem Koordinatensystem der Verstelleinheit für die Ausleuchtvorrichtung andererseits eine Transformationsmatrix ermittelt werden, um die beiden erzeugten Koordinatensysteme miteinander in Einklang bringen zu können. Eine solche Transformationsmatrix ist zwingend notwendig, um eine entsprechende Regelstrecke zur Verfügung stellen zu können. Bei bekannten Ausleuchtvorrichtungen ist die Kalibration, also die Erzeugung einer solchen Transformationsmatrix, mit sehr hohem Aufwand besetzt.

Es ist daher Aufgabe der vorliegenden Erfindung, die voranstehend beschriebenen Nachteile zumindest teilweise zu beheben. Insbesondere ist es Aufgabe der vorliegenden Erfindung, in einfacher und kostengünstiger Weise insbesondere eine zeitsparendes, bevorzugt ein automatisierbares Kalibrierverfahren zur Verfügung zu stellen.

Voranstehende Aufgabe wird gelöst durch ein erfindungsgemäßes Verfahren mit den Merkmalen des Anspruchs 1 sowie durch ein erfindungsgemäßes Ausleuchtsystem mit den Merkmalen des Anspruchs 12. Weitere Merkmale und Details der Erfindung ergeben sich aus den Unteransprüchen, der Beschreibung und den Zeichnungen. Dabei gelten Merkmale und Details, die im Zusammenhang mit dem erfindungsgemäßen Verfahren beschrieben sind, selbstverständlich auch im Zusammenhang mit dem erfindungsgemäßen Ausleuchtsystem und jeweils umgekehrt, sodass bezüglich der Offenbarung zu den einzelnen Erfindungsaspekten stets wechselseitig Bezug genommen wird bzw. werden kann.

Ein erfindungsgemäßes Verfahren dient der Kalibrierung einer Sensorvorrichtung für die Regelung der Ausrichtung einer Ausleuchtvorrichtung. Dabei dient es der Veränderung der Position eines durch die Ausleuchtvorrichtung in einer Ausleuchtrichtung erzeugten Ausleuchtbereichs. Ein erfindungsgemäßes Verfahren weist die folgenden Schritte auf:
a) Erzeugen eines von der Ausleuchtvorrichtung in der Ausleuchtrichtung ausgehenden Lichtpunktes auf einer Kalibrierfläche,
b) Definieren eines ersten Kalibrierpunktes auf der Kalibrierfläche für die Ausleuchtvorrichtung, insbesondere in einem Koordinatensystem der Ausleuchtvorrichtung,
c) Erkennen der Position des Lichtpunktes auf der Kalibrierfläche mit der Sensorvorrichtung, insbesondere in einem Koordinatensystem der Sensorvorrichtung,
d) Bestimmen des Abstandes zwischen dem Kalibrierpunkt und der erkannten Position des Lichtpunktes,
e) Verändern der Ausrichtung der Ausleuchtvorrichtung,
f) Wiederholtes Durchführen der Schritte c) bis e), bis zum Erreichen eines Abbruchkriteriums,
g) Definieren des ersten Kalibrierpunktes und der erkannten Position des Lichtpunktes auf der Kalibrierfläche als ein Korrespondenzpunkt zur Beziehung zwischen dem Koordinatensystem der Sensorvorrichtung und dem Koordinatensystem der Ausleuchtvorrichtung,
h) Definieren wenigstens eines weiteren Kalibrierpunktes,
i) Durchführen der Schritte c) bis g) für wenigstens einen weiteren Kalibrierpunkt.

Ein erfindungsgemäßes Verfahren dient also der Kalibrierung der Sensorvorrichtung, vorzugsweise in automatisierbarer Art. Dabei wird eine Korrespondenz zwischen der Erkennungsmöglichkeit der Sensorvorrichtung und der Beeinflussungsmöglichkeit der Ausleuchtvorrichtung festgestellt. Kern der vorliegenden Erfindung ist es, einen Lichtpunkt zur Verfügung zu stellen. Unter einem Lichtpunkt ist dabei insbesondere ein Laserlichtpunkt zu verstehen. Grundsätzlich sind jedoch auch andere Lichtpunkte im Rahmen der vorliegenden Erfindung denkbar. Von Vorteil ist es, wenn die geometrische Erstreckung des Lichtpunktes möglichst klein gehalten wird. Aufgrund des vorbestimmten Abstandes zwischen der Ausleuchtvorrichtung und der Kalibrierfläche ist dementsprechend eine Lichtquelle für die Erzeugung des Lichtpunktes von Vorteil, welche eine möglichst geringe Auffächerung bzw. eine möglichst große Fokussierung des erzeugten Lichtstrahls auf die Kalibrierfläche ermöglicht.

Ein Kalibrierpunkt ist im Sinne der vorliegenden Erfindung ein virtueller Punkt auf der Kalibrierfläche. Er wird als realer Punkt der Kalibrierfläche definiert und ist dementsprechend im realen Koordinatensystem definiert. Dieser erste Kalibrierpunkt dient als Vergleichspunkt für die erkannten Positionen des Lichtpunktes auf der Kalibrierfläche.

Erfindungsgemäß werden einzelne Schritte schleifenweise wiederholt. So wird in einer ersten kleinen Schleife der erste Kalibrierpunkt immer wieder mit dem Lichtpunkt verglichen und anschließend nach der Bestimmung des Abstandes zwischen dem Kalibrierpunkt und dem Lichtpunkt die Ausrichtung der Ausleuchtvorrichtung verändert. Selbstverständlich kann der Schritt der Veränderung der Ausleuchtvorrichtung bereits den vorangehend bestimmten Abstand zwischen dem Kalibrierpunkt und dem Lichtpunkt berücksichtigen. Damit ist es das Ziel, ein Abbruchkriterium zu erreichen, welches ein Ende für diese kleine Schleife zwischen Erkennen der Position des Lichtpunktes, Bestimmen des Abstandes und Verändern der Ausrichtung zur Verfügung stellt. Insbesondere wird durch wiederholtes Ausführen dieser drei Schritte eine Reduktion des bestimmten Abstandes zwischen dem Kalibrierpunkt und der erkannten Position des Lichtpunktes durchgeführt. Mit anderen Worten dient diese kleine Schleife dazu, eine Annäherung zwischen Lichtpunkt und Kalibrierpunkt zu erzielen. Dementsprechend befasst sich das Abbruchkriterium also vorzugsweise direkt, jedoch möglicherweise auch indirekt mit einer Minimierung des Abstandes zwischen dem Kalibrierpunkt und dem Lichtpunkt.

Wird das Abbruchkriterium erreicht, so werden der Kalibrierpunkt und die erkannte Position des Lichtpunktes als Korrespondenzpunkt der Ausleuchtvorrichtung und der Sensorvorrichtung gesetzt. Mit anderen Worten besteht nunmehr für diesen Punkt eine Korrespondenz in den unterschiedlichen Koordinatensystemen der Sensorvorrichtung und der Ausleuchtvorrichtung. Während der Lichtpunkt in seiner Position im Koordinatensystem der Sensorvorrichtung erkannt wird, besteht gleichzeitig eine entsprechende Korrespondenz zur Position der Ausleuchtvorrichtung. Diese Korrespondenz wirkt sich dahingehend aus, dass nunmehr die bestimmte Ausrichtung der Ausleuchtvorrichtung, also die Positionierung der Ausleuchtvorrichtung mit dem erreichten Abbruchkriterium, einen Lichtpunkt erzeugt, welcher mit dem definierten Kalibrierpunkt übereinstimmt bzw. unter Berücksichtigung des Abbruchkriteriums im Wesentlichen übereinstimmt. Dieser erste Korrespondenzpunkt stellt nunmehr eine Beziehung her zwischen dem Koordinatensystem der Ausleuchtvorrichtung einerseits und dem Koordinatensystem der Sensorvorrichtung andererseits. Somit kann der Korrespondenzpunkt Eingang finden in die notwendige Transformationsmatrix zwischen diesen beiden Koordinatensystemen der Sensorvorrichtung und der Ausleuchtvorrichtung. Während also der Kalibrierpunkt innerhalb des Koordinatensystems der Ausleuchtvorrichtung und der Lichtpunkt im Koordinatensystem der Sensorvorrichtung definiert ist, stellt der Korrespondenzpunkt eine Koordinatenpaarung jeweils in diesen beiden Koordinatensystemen dar, welche in der Realität auf den identischen oder im Wesentlichen identischen Punkt führen.

Um sicherzustellen, dass die Transformationsmatrix auch bei komplexen Koordinatensystemen ausreichende Eingabewerte beinhaltet, wird wenigstens ein weiterer Kalibrierpunkt gesetzt, für welchen die beschriebenen Schritte c) bis g) nochmals durchgeführt werden. Bevorzugt ist es, dass für wenigstens drei Kalibrierpunkte die Schritte c) bis g) insgesamt durchgeführt werden. Damit kann im dreidimensionalen Koordinatensystem auch eine dreidimensionale Transformationsmatrix erzeugt werden, um die erfindungsgemäß zur Verfügung stellbare Kalibrierung zwischen Sensorvorrichtung und Ausleuchtvorrichtung erzeugen zu können.

Unter einer Sensorvorrichtung ist im Sinne der vorliegenden Erfindung insbesondere eine Kameravorrichtung und/oder eine Kameraeinheit zu verstehen. Die Ausleuchtvorrichtung ist insbesondere eine Operationsleuchtvorrichtung, die zur Ausleuchtung eines Operationsausleuchtbereichs Einsatz finden soll. Dementsprechend ist der Ausleuchtbereich insbesondere ein Operationsausleuchtbereich.

Die Kalibrierfläche kann durch einen separaten Kalibrierkörper zur Verfügung gestellt werden. Jedoch ist es auch möglich, dass die Kalibrierfläche bereits z. B. durch einen vorhandenen Tisch, insbesondere einen Operationstisch, vorhanden ist. Die Kalibrierfläche ist vorzugsweise angepasst an eine ausreichende Reflexion des Lichtes, welches den Lichtpunkt ausbildet. Damit wird sichergestellt, dass der Lichtpunkt auch in der gewünschten und für das Verfahren notwendigen Weise von der Sensorvorrichtung wahrgenommen werden kann. Der Lichtpunkt ist das Ergebnis der Projektion eines Lichtstrahls mit vorzugsweise nur geringem Auffächerungsgrad. Insbesondere wird Laserlicht, vorzugsweise im Wesentlichen kohärentes Licht verwendet.

Weiter ist es möglich, dass für die Erkennung der Position eine Aufspaltung in das grundsätzliche Erkennen des Lichtpunktes, z. B. durch eine Farbkamera, und das Positionieren des erkannten Punktes, z. B. durch eine Tiefenkamera, erfolgen kann.

Ein Abbruchkriterium ist im Wesentlichen frei bestimmbar. So kann allein durch eine vorbestimmte Anzahl an Schleifenwiederholungen ein Abbruchkriterium zur Verfügung gestellt werden. Dabei handelt es sich also um eine maximale Iterationszahl der entsprechenden Schleife. Auch das Erreichen einer minimalen Abweichung und dementsprechend einer Korrespondenzgenauigkeit zwischen dem erkannten Lichtpunkt und dem zugehörigen Kalibrierpunkt ist ein Abbruchkriterium im Sinne der vorliegenden Erfindung.

Die Punktkorrespondenzen werden, wie in beschriebener Weise, für die Erzeugung der Transformationsmatrix zwischen Sensorvorrichtung und Ausleuchtvorrichtung verwendet. Um in einem dreidimensionalen Koordinatensystem eine entsprechend ausreichend bestimmte Transformationsmatrix zur Verfügung zu stellen, sind vorzugsweise insgesamt drei Kalibrierpunkte mit den entsprechenden Verfahrensschritten belegt worden.

Eine Veränderung der Ausrichtung der Ausleuchtvorrichtung erfolgt insbesondere durch ein Verändern des Winkels der Ausleuchtrichtung und/oder ein Verschieben der Ausleuchtrichtung. Bevorzugt ist es jedoch, wenn eine Parallelverschiebung der Ausleuchtvorrichtung zwischen den einzelnen Veränderungsschritten für die Ausleuchtvorrichtung hinsichtlich ihrer Ausrichtung erfolgt.

Ein erfindungsgemäßes Verfahren wird vorzugsweise nach der Montage des entsprechenden Ausleuchtsystems durchgeführt. Vorzugsweise wird es jedoch häufiger, z. B. mindestens einmal täglich, durchgeführt, um eine möglicherweise entstehende Verstellung der Sensorvorrichtung und/oder der Ausleuchtvorrichtung durch Rekalibration ausgleichen zu können. Ein erfindungsgemäßes Verfahren wird vorzugsweise automatisiert durchgeführt, sodass insbesondere Verfahrensdurchläufe für alle Iterationszahlen von weniger als ca. 1 Minute erzielbar werden.

Ein erfindungsgemäßes Verfahren lässt sich dahingehend weiterbilden, dass der Lichtpunkt in Form eines Laserlichtpunktes erzeugt wird. Insbesondere wird ein Laser mit kohärentem Licht verwendet. Dies erlaubt es, auch größere Abstände zwischen der Ausleuchtvorrichtung und der Kalibrierfläche zuzulassen. Gleichzeitig wird eine möglichst punktförmige Ausbildung des Lichtpunktes gewährleistet, um auch in definierter Weise möglichst genau den Abstand zwischen der Position des Lichtpunktes und dem Kalibrierpunkt definieren zu können. Insbesondere folgt eine Anpassung der Oberfläche der Kalibrierfläche an die Wellenlänge des Lasers und/oder die Art der Sensorvorrichtung. Dabei handelt es sich insbesondere um eine Anpassung hinsichtlich der Reflexionseigenschaften der Kalibrierfläche.

Vorteilhaft ist es ebenfalls, wenn bei einem erfindungsgemäßen Verfahren der Schritt des Erkennens der Position des Lichtpunktes auf der Kalibrierfläche auf zwei Sensoreinheiten der Sensorvorrichtung aufgeteilt ist. Dabei erkennt eine erste Sensoreinheit den Lichtpunkt auf der Kalibrierfläche und eine zweite Sensoreinheit bestimmt den Ort des erkannten Lichtpunktes auf der Kalibrierfläche. Die erste Sensoreinheit kann dabei z. B. eine Farbkamera und die zweite Sensoreinheit z. B. eine Tiefenkamera sein. Auch bereits bestehende Ausleuchtsysteme können auf diese Weise nachgerüstet werden. So kann es vorteilhaft sein, wenn bereits bei einer vorhandenen Tiefenkamera durch die Nachrüstung einer Farbkamera die Vorrichtungsgegebenheiten für die Durchführung eines erfindungsgemäßen Verfahrens nachgerüstet werden. Darüber hinaus können die jeweiligen Sensoreinheiten an die spezifische Aufgabe angepasst sein. Insbesondere ist es möglich, die zweite Sensoreinheit auch für die Durchführung eines Regelverfahrens bei einer Gestensteuerung während der Verwendung der Ausleuchtvorrichtung einzusetzen. Somit kann ein Teil der grundsätzlich notwendigen Regelungseinheiten, nämlich die zweite Sensoreinheit, auch für ein erfindungsgemäßes Kalibrierverfahren zur Verfügung gestellt werden. Dies reduziert die Komplexität der Vorrichtung und die damit einhergehenden Kosten.

Ebenfalls von Vorteil kann es sein, wenn bei einem erfindungsgemäßen Verfahren als Abbruchkriterium wenigstens eines der folgenden eingesetzt wird:
- Vorgabe einer maximalen Wiederholungszahl
- Vorgabe eines maximalen Abstandes zwischen der erkannten Position des Lichtpunktes auf der Kalibrierfläche mit dem Kalibrierpunkt

Es wird also vorzugsweise als Abbruchkriterium z. B. ein Genauigkeitswert in Form eines vordefinierten ε vorgegeben. Damit wird ein maximaler Abstand zwischen der erkannten Position des Lichtpunktes und dem Kalibrierpunkt vordefiniert, welcher im Wesentlichen als Identität zwischen der erkannten Position des Lichtpunktes und dem Kalibrierpunkt und dementsprechend als Korrespondenzpunkt gesetzt wird. Selbstverständlich können die beiden voranstehend beschriebenen Abbruchkriterien auch miteinander kombiniert werden, sodass eine maximale Wiederholungszahl eine Unendlichkeitsschleife des Verfahrens verhindert. Gleichzeitig muss jedoch nicht die maximale Wiederholungszahl erreicht werden, wenn bereits vorher eine Reduktion des Abstandes zwischen der erkannten Position des Lichtpunktes und dem Kalibrierpunkt und dem vordefinierten maximalen Abstand erfolgen konnte. Somit kann einerseits der Genauigkeitsgrad der Kalibrierung vorgegeben werden, während allgemein eine Zeitobergrenze für die Durchführung des Verfahrens definierbar wird. Die Vorgabe kann dabei allgemein oder auch spezifisch für jede Kalibrierung durchgeführt werden. Insbesondere sind solche Vorgabewerte zumindest grundsätzlich allgemein gespeichert, sodass die Durchführung des Verfahrens automatisiert erfolgen kann.

Vorteilhaft ist es darüber hinaus, wenn bei einem erfindungsgemäßen Verfahren die Veränderung der Ausrichtung der Ausleuchtvorrichtung nach einem vorgegebenen Muster erfolgt. Mit anderen Worten sind die Veränderungsbewegungen der Ausleuchtvorrichtung durch dieses Muster vorgegeben. Es erfolgt also eine zielgerichtete Veränderung der Ausrichtung, z. B. durch definierte Parallelverschiebung und/oder durch definiertes Verändern des Winkels der Ausleuchtrichtung. Das Muster ist insbesondere abhängig von der Zahl der Wiederholungen, sodass für jeden Iterationsschritt eine Veränderung des Musters vorgenommen werden kann. Die Veränderung des Musters zielt dabei insbesondere auf eine Erhöhung der Genauigkeit bzw. eine Reduktion des Abstandes zwischen den erzeugten Lichtpunkten und dem Kalibrierpunkt hin. Damit wird durch die Vorgabe eines Musters eine noch leichtere Automatisierung eines erfindungsgemäßen Kalibrierverfahrens möglich.

Ebenfalls von Vorteil ist es, wenn bei einem Verfahren gemäß dem voranstehenden Absatz es sich bei dem Muster um ein Suchgitter, insbesondere um ein 3x3x3 Suchgitter, mit gleichen oder im Wesentlichen gleichen Abständen der einzelnen Gitterpunkte handelt. Insbesondere ist das Suchgitter also ein dreidimensionales Suchgitter. Dementsprechend erfolgt die Veränderung der Ausrichtung der Ausleuchtvorrichtung auf insgesamt 27 Positionen. Dies entspricht einer Iterationszahl von 27 Wiederholungen für diese kleine Schleife, um in einem ersten Schritt einen Abstand zwischen dem erkannten Lichtpunkt und dem Kalibrierpunkt zu reduzieren. Insbesondere erfolgt eine Parallelverschiebung der Ausleuchtrichtung zwischen diesen einzelnen Punkten. Final wird derjenige Punkt ausgewählt, welcher den geringsten Abstand als Lichtpunkt zum Kalibrierpunkt erzeugt. Dieser ausgewählte Punkt des 3x3x3 Suchgitters wird als Ausgangspunkt für die nachfolgenden Iterationswege und dementsprechend für das nachfolgende Suchgitter ermöglicht, wie dies nachfolgend noch näher erläutert wird.

Bei einem erfindungsgemäßen Verfahren gemäß dem voranstehenden Absatz ist es weiter möglich, wenn nach Abfahren aller Gitterpunkte des Suchgitters derjenige Gitterpunkt mit der zugehörigen Position des Lichtpunktes mit dem geringsten Abstand zum Kalibrierpunkt als Ausgangspunkt für die Wiederholung mit dem nächsten Suchgitter, insbesondere mit reduziertem Abstand der Gitterpunkte, ausgewählt wird. Somit kann die Iteration mit immer feineren Suchgittern durchgeführt werden, wobei für jedes neue Suchgitter mit reduziertem Abstand, insbesondere halbiertem Abstand der Gitterpunkte, eine höhere Genauigkeit erzeugt werden kann. Als Ausgangspunkt für jedes neue Suchgitter wird derjenige Punkt des alten Suchgitters gewählt, welcher den geringsten Abstand zum Kalibrierpunkt zur Verfügung stellen konnte. Damit wird die gesamte Iteration mit steigernder Genauigkeit eine Reduktion des Abstandes der erzeugten Lichtpunkte zum Kalibrierpunkt gewährleisten und damit zum erfindungsgemäß zu bestimmenden Korrespondenzpunkt führen.

Ein weiterer Vorteil ist erzielbar, wenn bei einem erfindungsgemäßen Verfahren die Schritte a) bis i) insgesamt für wenigstens drei Kalibrierpunkte durchgeführt werden, welche alle in einer Ebene liegen, in welcher sich die Kalibrierfläche erstreckt. Dementsprechend kann in einem dreidimensionalen System auch eine Anzahl von drei Korrespondenzpunkten erzeugt werden, die wiederum eine ausreichende Datenbasis für die Erzeugung einer Transformationsmatrix zwischen Sensorvorrichtung und Ausleuchtvorrichtung bieten. Selbstverständlich können jedoch auch mehr als drei Kalibrierpunkte in einem erfindungsgemäßen Verfahren Einsatz finden. Die drei Kalibrierpunkte definieren eindeutig die Kalibrierfläche. Sämtliche weiteren Kalibrierpunkte liegen ebenfalls in dieser Kalibrierfläche. Sie erhöhen die Genauigkeit eines erfindungsgemäßen Verfahrens weiter, da insbesondere eine Plausibilitätsprüfung bei der Erzeugung der Transformationsmatrix zwischen Sensorvorrichtung und Ausleuchtvorrichtung berücksichtigt werden kann.

Vorteilhaft ist es ebenfalls, wenn bei einem erfindungsgemäßen Verfahren alle definierten Korrespondenzpunkte als Eingangswerte für eine nachfolgende Regelung der Ausrichtung der Ausleuchtvorrichtung gespeichert und/oder an eine Kontrolleinheit zur Durchführung der Regelung dieser Ausrichtung übermittelt werden. Mit anderen Worten erfolgt eine Übergabe bzw. eine direkte Berechnung der bereits beschriebenen Transformationsmatrix. Diese Transformationsmatrix erlaubt eine Transformation zwischen dem Koordinatensystem der Sensorvorrichtung und dem Koordinatensystem der Ausleuchtvorrichtung. Sie beruht auf einer Korrespondenz im real vorhandenen dreidimensionalen Koordinatensystem der Kalibrierfläche. Mit anderen Worten erzeugt die Kalibration direkt oder indirekt die Transformationsmatrix. Dabei kann ein Teil des erfindungsgemäßen Verfahrens die Erstellung der Transformationsmatrix sein. Jedoch ist es grundsätzlich ausreichend, wenn die definierten Korrespondenzpunkte einer nachfolgenden Kontrolleinheit zur Verfügung gestellt werden, welche wiederum in der Lage ist, die notwendige Transformationsmatrix zu erzeugen.

Vorteilhaft ist es weiter, wenn bei einem erfindungsgemäßen Verfahren der für den ersten Kalibrierpunkt gefundene Korrespondenzpunkt für die Durchführung der Schritte für den wenigstens einen weiteren Kalibrierpunkt verwendet wird. Das bedeutet, dass die nachfolgenden Kalibrierpunkte schneller zu einem Korrespondenzpunkt führen, da bereits eine erste Grundtendenz des Zusammenhangs zwischen Ausleuchtvorrichtung und Sensorvorrichtung besteht. Mit anderen Worten können für die weiteren Kalibrierpunkte die bereits gefundenen Ergebnisse, insbesondere die bereits gefundenen Korrespondenzpunkte zur Verbesserung und zur Beschleunigung des Verfahrens Verwendung finden. Damit kann für die nachfolgenden Iterationen für weitere Kalibrierpunkte eine Reduktion der Iterationszahl erreicht werden und damit die Durchführungsdauer und die maximale Iterationszahl für ein erfindungsgemäßes Verfahren reduziert werden.

Vorteilhaft ist es darüber hinaus, wenn bei einem erfindungsgemäßen Verfahren vor Erzeugen des Lichtpunktes ein Kalibrierkörper zur Verfügung gestellt wird, welcher die Kalibrierfläche aufweist. Dies ist insbesondere dann der Fall, wenn z. B. in einem Operationsaal kein Operationstisch vorhanden ist. Auch kann dies von Vorteil sein, wenn das entsprechende Objekt, z. B. ein Operationstisch, schlechte Reflexionswerte aufweist. Der Kalibrierkörper ist z. B. aus Metall oder aber auch einem anderen Material gefertigt, um insbesondere bei Laserlicht für einen Laserlichtpunkt eine möglichst vorteilhafte Reflexion mit sich zu bringen.

Ebenfalls Gegenstand der vorliegenden Erfindung ist ein Ausleuchtsystem mit einer Ausleuchtvorrichtung, einer Sensorvorrichtung und einer Kontrolleinheit für die Regelung der Ausrichtung der Ausleuchtvorrichtung zur Veränderung der Position des durch die Ausleuchtvorrichtung in einer Ausleuchtrichtung erzeugten Ausleuchtbereichs, wobei die Sensorvorrichtung und die Kontrolleinheit für die Ausleuchtung eines erfindungsgemäßen Verfahrens ausgebildet sind. Dementsprechend bringt ein erfindungsgemäßes Ausleuchtsystem die gleichen Vorteile mit sich, wie sie ausführlich mit Bezug auf ein erfindungsgemäßes Verfahren erläutert worden sind. Vorzugsweise weist ein erfindungsgemäßes Ausleuchtsystem eine Lichtquelle auf, um den beschriebenen Lichtpunkt zu erzeugen. Dabei handelt es sich insbesondere um eine Laserlichtquelle. Diese muss nicht permanent in/an der Ausleuchtausrichtung befestigt/integriert sein, sondern kann auch nur für die Kalibrierung angebaut werden.

Weitere Vorteile, Merkmale und Einzelheiten der Erfindung ergeben sich aus der Beschreibung, in der unter Bezugnahme auf die Zeichnungen Ausführungsbeispiele der Erfindung im Einzelnen beschriebenen sind. Dabei können die in den Ansprüchen und der Beschreibung erwähnten Merkmale jeweils einzeln für sich oder in beliebiger Kombination erfindungswesentlich sein. Es zeigen schematisch:
- Figur 1: eine Ausführungsform eines erfindungsgemäßen Ausleuchtsystems,
- Figur 2: eine Korrelation zwischen der Ausleuchtvorrichtung und der Kalibrierfläche,
- Figur 3: eine Korrelation zwischen einem Suchgitter und der Ausleuchtvorrichtung,
- Figur 4: ein erstes Suchgitter,
- Figur 5: ein zweites Suchgitter und
- Figur 6: die schematische Darstellung der einzelnen Verfahrensschritte.

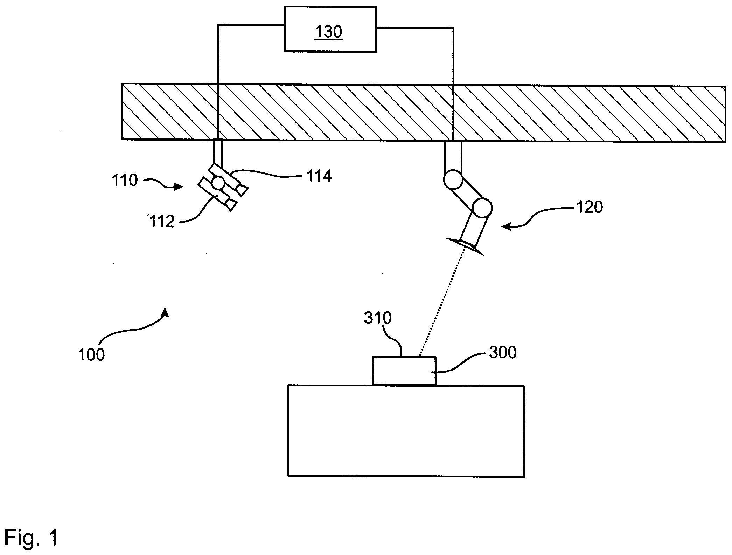

In der Figur 1 ist eine Ausführungsform eines erfindungsgemäßen Ausleuchtsystems 100 dargestellt. An der Decke eines Operationssaales ist eine Ausleuchtvorrichtung 120 bewegbar an einem Roboterarm befestigt. Darüber hinaus ist eine Sensorvorrichtung 110 mit zwei Sensoreinheiten 112 und 114 vorgesehen. Die erste Sensoreinheit 112 ist dabei als Farbkamera und die zweite Sensoreinheit 114 vorzugsweise als Tiefenkamera ausgebildet.

Weiter ist zu erkennen, dass ausgehend von der Ausleuchtvorrichtung 120 (gepunkteter Lichtstrahl) ein Lichtpunkt auf einer Kalibrierfläche 310 eines Kalibrierkörpers 300 erzeugt wird. Darüber hinaus ist eine signalkommunizierende Verbindung zwischen Ausleuchtvorrichtung 120 und einer Kontrolleinheit 130 vorgesehen. Diese Kontrolleinheit 130 steht ebenfalls in signalkommunizierender Verbindung mit der Sensorvorrichtung 110. Um nun die Sensorvorrichtung 110 mit der Ausleuchtvorrichtung 120 zu kalibrieren, erfolgt ein Kalibrationsverfahren gemäß der vorliegenden Erfindung.

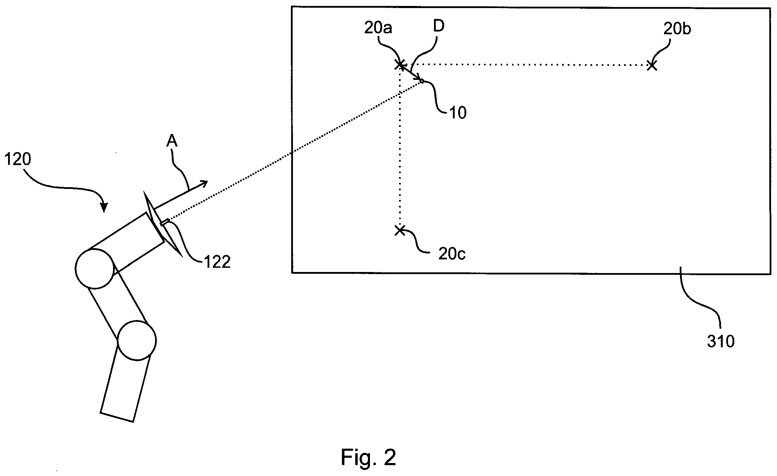

In Figur 2 ist eine Draufsicht auf die Kalibrierfläche 310 gezeigt. Hier werden drei Kalibrierpunkte 20a, 20b und 20c in der Kalibrierfläche 310 definiert. Gleichzeitig gibt Figur 2 im Wesentlichen das Bild wieder, welches von der Sensorvorrichtung 110 erkannt wird. Schematisch ist links die Ausleuchtvorrichtung 120 dargestellt, deren Ausleuchtrichtung A ausgehend von der Lichtquelle 122 des Lichtpunktes 10 parallel eine Erstreckung aufweist. Der Lichtpunkt 10 wird von der Sensorvorrichtung 110 wahrgenommen und mit dem vordefinierten nächsten ersten Kalibrierpunkt 20a verglichen. Dabei wird der Abstand D zwischen dem ersten Kalibrierpunkt 20a und dem Lichtpunkt 10 definiert bzw. bestimmt.

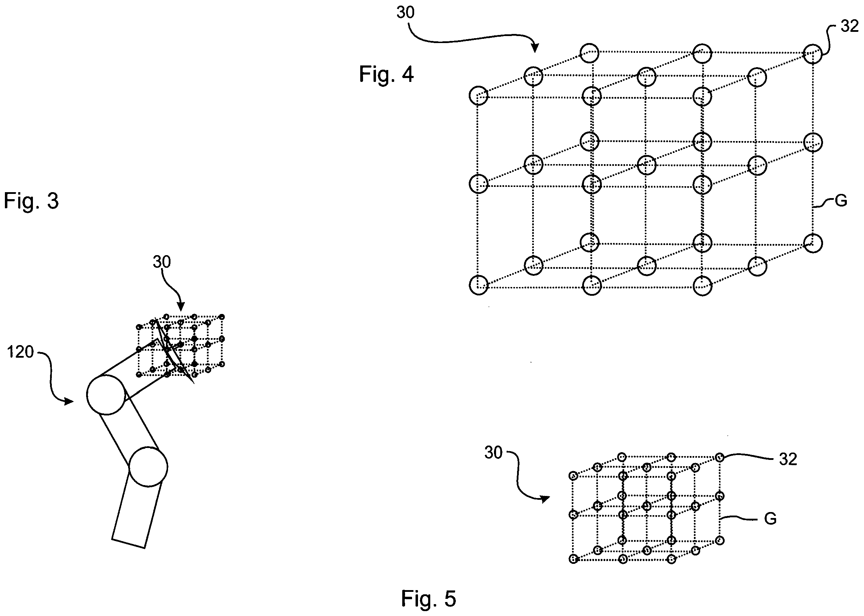

Anschließend an diese Bestimmung erfolgt eine definierte Veränderung der Ausrichtung der Ausleuchtvorrichtung 120. Diese kann z. B. entlang eines vordefinierten Musters erfolgen. Ein solches Muster kann ein Suchgitter 30 sein, wie es die Figur 3 zeigt. Das Suchgitter 30 ist eine 3x3x3 Matrix, welche sich um den Startpunkt, erstreckt. Dabei wird die Ausleuchtvorrichtung 120 hinsichtlich ihrer Ausleuchtrichtung A parallel verschoben auf alle 27 Punkte dieser 3x3x3 Matrix dieses Suchgitters 30. Anschließend wird von all diesen 27 erzeugten Lichtpunkten 10 der minimale Abstand D zum ersten Kalibrierpunkt 20a bestimmt. Dieser Punkt ist nun Ausgangspunkt für das nachfolgende Suchgitter 30.

Die einzelnen Suchgitter 30 in den Iterationsschritten können sich vorzugsweise verkleinern. So zeigt Figur 4 z. B. ein erstes Suchgitter 30 mit einer Vielzahl von Gitterpunkten 32 und einem ersten Abstand G zwischen den Gitterpunkten 32. Wurden all diese 27 Gitterpunkte 32 abgefahren, konnte der minimale Abstand des Lichtpunktes 10 zum ersten Kalibrierpunkt 20a bestimmt werden. Anschließend wird der korrespondierende Punkt des Suchgitters 30 als Ausgangspunkt für die nachfolgende Iteration gewählt. Für das nachfolgende Suchgitter 30 wird vorzugsweise ein reduzierter Abstand G zwischen den Gitterpunkten 32 gewählt, wie dies z. B. die Figur 5 zeigt. In Summe kann bis zum Erreichen eines minimalen Abstandes D zwischen Lichtpunkt 10 und ersten Kalibrierpunkt 20a eine Minimierung des Abstandes D erzielt werden.

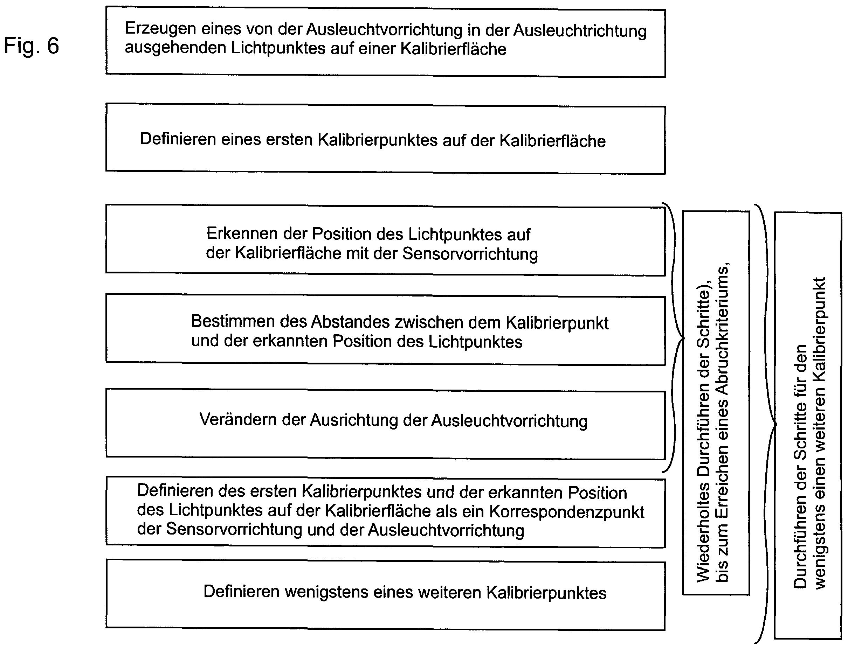

Wie z. B. der Figur 6 zu entnehmen ist, wird eine Vielzahl von zwei Schleifen durchgeführt. Die kleine Schleife ist dabei die Durchführung einer Iteration für einen Kalibrierpunkt bis zum Erzeugen eines ersten Korrespondenzpunktes. Die große Schleife ist dabei die Durchführung dieser kleinen Schleife für insgesamt wenigstens zwei, vorzugsweise wenigstens drei Kalibrierpunkte. Dementsprechend werden insbesondere drei Korrespondenzpunkte gefunden, welche nachfolgend für die Kontrolleinheit 130 und/oder in der Kontrolleinheit 130 als Korrespondenzmatrix zur Verfügung gestellt werden können. Diese Transformationsmatrix als Korrespondenzmatrix dient der Übersetzung der erkannten Punkte durch die Sensorvorrichtung 110 in das entsprechende Koordinatensystem zur Steuerung der Ausleuchtvorrichtung 120.

Die voranstehende Erläuterung der Ausführungsformen beschreibt die vorliegende Erfindung ausschließlich im Rahmen von Beispielen. Selbstverständlich können einzelne Merkmale der Ausführungsformen, sofern technisch sinnvoll, frei miteinander kombiniert werden, ohne den Rahmen der vorliegenden Erfindung zu verlassen.

### BEZUGSZEICHENLISTE

- 10: Lichtpunkt
- 20a: erster Kalibrierpunkt
- 20b: zweiter Kalibrierpunkt
- 20c: dritter Kalibrierpunkt
- 30: Suchgitter
- 32: Gitterpunkt

- 100: Ausleuchtsystem
- 110: Sensorvorrichtung
- 112: Sensoreinheit
- 114: Sensoreinheit
- 120: Ausleuchtvorrichtung
- 122: Lichtquelle des Lichtpunkts
- 130: Kontrolleinheit

- 300: Kalibrierkörper
- 310: Kalibrierfläche

- A: Ausleuchtrichtung
- D: Abstand zwischen dem Kalibrierpunkt und der Position des Lichtpunkts
- G: Abstand zwischen den Gitterpunkten des Suchgitters

## Patentansprüche

1. Verfahren für die Kalibrierung einer Sensorvorrichtung (11 0) für die Regelung der Ausrichtung einer Ausleuchtvorrichtung (120) zur Veränderung der Position eines durch die Ausleuchtvorrichtung (120) in einer Ausleuchtrichtung (A) erzeugten Ausleuchtbereichs, aufweisend die folgenden Schritte:
a) Erzeugen eines von der Ausleuchtvorrichtung (120) in der Ausleuchtrichtung (A) ausgehenden Lichtpunktes (10) auf einer Kalibrierfläche (310),
b) Definieren eines ersten Kalibrierpunktes (20a) auf der Kalibrierfläche (310) für die Ausleuchtvorrichtung (120),
c) Erkennen der Position des Lichtpunktes (10) auf der Kalibrierfläche (310) mit der Sensorvorrichtung (110),
d) Bestimmen des Abstandes (D) zwischen dem Kalibrierpunkt (20a) und der erkannten Position des Lichtpunktes (1 0),
e) Verändern der Ausrichtung der Ausleuchtvorrichtung (120),
f) Wiederholtes Durchführen der Schritte c) bis e), bis zum Erreichen eines Abbruchkriteriums,
g) Definieren des ersten Kalibrierpunktes (20a) und der erkannten Position des Lichtpunktes (10) auf der Kalibrierfläche (310) als ein Korrespondenzpunkt zur Beziehung zwischen dem Koordinatensystem der Sensorvorrichtung (110) und dem Koordinatensystem der Ausleuchtvorrichtung (120),
h) Definieren wenigstens eines weiteren Kalibrierpunktes (20b, 20c),
i) Durchführen der Schritte c) bis g) für den wenigstens einen weiteren Kalibrierpunkt (20b, 20c).

2. Verfahren nach Anspruch 1, **dadurch gekennzeichnet, dass** der Lichtpunkt (10) in Form eines Laserlichtpunktes erzeugt wird.

3. Verfahren nach einem der vorangegangenen Ansprüche, **dadurch gekennzeichnet, dass** der Schritt des Erkennens der Position des Lichtpunktes (10) auf der Kalibrierfläche (310) auf zwei Sensoreinheiten (112, 114) der Sensorvorrichtung (110) aufgeteilt ist, wobei eine erste Sensoreinheit (112) den Lichtpunkt (10) auf der Kalibrierfläche (310) erkennt und eine zweite Sensoreinheit (114) den Ort des erkannten Lichtpunktes (10) auf der Kalibrierfläche (310) bestimmt.

4. Verfahren nach einem der vorangegangenen Ansprüche, **dadurch gekennzeichnet, dass** als Abbruchkriterium wenigstens eines der folgenden eingesetzt wird:
- Vorgabe einer maximalen Wiederholungszahl
- Vorgabe eines maximalen Abstandes (D) zwischen der erkannten Position des Lichtpunktes (10) auf der Kalibrierfläche (310) und dem Kalibrierpunkt (20a, 20b, 20c).

5. Verfahren nach einem der vorangegangenen Ansprüche, **dadurch gekennzeichnet, dass** die Veränderung der Ausrichtung der Ausleuchtvorrichtung (120) nach einem vorgegebenen Muster erfolgt.

6. Verfahren nach Anspruch 5, **dadurch gekennzeichnet, dass** es sich bei dem Muster um ein Suchgitter (30), insbesondere um ein 3x3x3 Suchgitter (30), mit gleichen oder im Wesentlichen gleichen Abständen (G) der einzelnen Gitterpunkte (32) handelt.

7. Verfahren nach Anspruch 6, **dadurch gekennzeichnet, dass** nach Abfahren aller Gitterpunkte (32) des Suchgitters (30) derjenige Gitterpunkt (32) mit der zugehörigen Position des Lichtpunkts (10) mit dem geringsten Abstand zum Kalibrierpunkt (20a, 20b, 20c) als Ausgangspunkt für die Wiederholung mit dem nächsten Suchgitter (30), insbesondere mit reduziertem Abstand (G) der Gitterpunkte (32), ausgewählt wird.

8. Verfahren nach einem der vorangegangenen Ansprüche, **dadurch gekennzeichnet, dass** die Schritte a) bis i) insgesamt für wenigstens drei Kalibrierpunkte (20a, 20b, 20c) durchgeführt werden, welche alle in einer Ebene liegen, in welcher sich die Kalibrierfläche (310) erstreckt.

9. Verfahren nach einem der vorangegangenen Ansprüche, **dadurch gekennzeichnet, dass** alle definierten Korrespondenzpunkte als Eingangswerte für eine nachfolgende Regelung der Ausrichtung der Ausleuchtvorrichtung (120) gespeichert und/oder an eine Kontrolleinheit (130) zur Durchführung der Regelung dieser Ausrichtung übermittelt werden.

10. Verfahren nach einem der vorangegangenen Ansprüche, **dadurch gekennzeichnet, dass** der für den ersten Kalibrierpunkt (20a) gefundene Korrespondenzunkt für die Durchführung der Schritte für den wenigstens einen weiteren Kalibrierpunkt (20b, 20c) verwendet wird.

11. Verfahren nach einem der vorangegangenen Ansprüche, **dadurch gekennzeichnet, dass** vor Erzeugen des Lichtpunkts (10) ein Kalibrierkörper (300) zur Verfügung gestellt wird, welcher die Kalibrierfläche (310) aufweist.

12. Ausleuchtsystem (100) mit einer Ausleuchtvorrichtung (120), einer Sensorvorrichtung (110) und einer Kontrolleinheit (130) für die Regelung der Ausrichtung der Ausleuchtvorrichtung (120) zur Veränderung der Position des durch die Ausleuchtvorrichtung (120) in einer Ausleuchtrichtung (A) erzeugten Ausleuchtbereichs, wobei die Sensorvorrichtung (110) und die Kontrolleinheit (130) für die Ausführung eines Verfahrens mit den Merkmalen eines Ansprüche 1 bis 11 ausgebildet sind.

## Claims

1. A method for calibrating a sensor device (110) for regulating the alignment of an illumination device (120) for modifying the position of an illumination region generated by the illumination device (120) in an illumination direction (A), having the following steps:
a) generating a light spot (10), emanating from the illumination device (120) in the illumination direction (A), on a calibration surface (310),
b) defining a first calibration point (20a) on the calibration surface (310) for the illumination device (120),
c) identifying the position of the light spot (10) on the calibration surface (310) with the sensor device (110),
d) determining the distance (D) between the calibration point (20a) and the identified position of the light spot (10),
e) modifying the alignment of the illumination device (120),
f) repeatedly performing steps c) to e) until a stop criterion is reached,
g) defining the first calibration point (20a) and the identified position of the light spot (10) on the calibration surface (310) as a correspondence point for the relationship between the coordinate system of the sensor device (110) and the coordinate system of the illumination device (120),
h) defining at least one further calibration point (20b, 20c),
i) performing steps c) to g) for the at least one further calibration point (20b, 20c).

2. A method according to claim 1, **characterised in that** the light spot (10) is generated in the form of a laser light spot.

3. A method according to one of the preceding claims, **characterised in that** the step of identifying the position of the light spot (10) on the calibration surface (310) is split between two sensor units (112, 114) of the sensor device (110), wherein a first sensor unit (112) identifies the light spot (10) on the calibration surface (310), and a second sensor unit (114) determines the location of the identified light spot (10) on the calibration surface (310).

4. A method according to one of the preceding claims, **characterised in that** at least one of the following is used as the stop criterion:
- specification of a maximum number of repetitions
- specification of a maximum distance (D) between the identified position of the light spot (10) on the calibration surface (310) and the calibration point (20a, 20b, 20c).

5. A method according to one of the preceding claims, **characterised in that** the modification of the alignment of the illumination device (120) is effected according to a predetermined pattern.

6. A method according to claim 5, **characterised in that** the pattern is a search grid (30), in particular a 3x3x3 search grid (30), with the same or substantially the same distances (G) between the individual grid points (32).

7. A method according to claim 6, **characterised in that** after covering all the grid points (32) of the search grid (30) that grid point (32) having the associated position of the light spot (10) with the shortest distance to the calibration point (20a, 20b, 20c) is selected as a starting point for the repetition with the next search grid (30), in particular with a reduced distance (G) between the grid points (32).

8. A method according to one of the preceding claims, **characterised in that** steps a) to i) are performed in total for at least three calibration points (20a, 20b, 20c), all of which lie in a plane in which the calibration surface (310) extends.

9. A method according to one of the preceding claims, **characterised in that** all the defined correspondence points are stored as input values for subsequent regulation of the alignment of the illumination device (120) and/or are transmitted to a control unit (130) for performing the regulation of this alignment.

10. A method according to one of the preceding claims, **characterised in that** the correspondence point found for the first calibration point (20a) is used for performing the steps for the at least one further calibration point (20b, 20c).

11. A method according to one of the preceding claims, **characterised in that** prior to generating the light spot (10) a calibration body (300) is made available that has the calibration surface (310).

12. An illumination system (100) having an illumination device (120), a sensor device (110) and a control unit (130) for regulating the alignment of the illumination device (120) for modifying the position of the illumination region generated by the illumination device (120) in an illumination direction (A), wherein the sensor device (110) and the control unit (130) are configured to carry out a method having the features of one of claims 1 to 11.

## Revendications

1. Procédé d'étalonnage d'un dispositif de détection (110) servant à réguler l'orientation d'un dispositif d'éclairage (120) afin de modifier la position d'une zone d'éclairage produite par le dispositif d'éclairage (120) dans une direction d'éclairage (A), comportant les étapes suivantes :
a) production sur une surface d'étalonnage (310) d'un point lumineux (10) émis par le dispositif d'éclairage (120) dans la direction d'éclairage (A),
b) définition d'un premier point d'étalonnage (20a) sur la surface d'étalonnage (310) pour le dispositif d'éclairage (120),
c) détection de la position du point lumineux (10) sur la surface d'étalonnage (310) au moyen du dispositif de détection (110),
d) détermination de la distance (D) entre le point d'étalonnage (20a) et la position détectée du point lumineux (10),
e) modification de l'orientation du dispositif d'éclairage (120),
f) répétition des étapes c) à e) jusqu'à atteindre un critère d'interruption,
g) définition du premier point d'étalonnage (20a) et de la position détectée du point lumineux (10) sur la surface d'étalonnage (310) comme point de correspondance pour la mise en relation du système de coordonnées du dispositif de détection (110) avec le système de coordonnées du dispositif d'éclairage (120),
h) définition d'au moins un autre point d'étalonnage (20b, 20c),
i) exécution des étapes c) à g) pour ledit au moins un autre point d'étalonnage (20b, 20c).

2. Procédé selon la revendication 1, **caractérisé en ce que** le point lumineux (10) est produit sous la forme d'un point lumineux laser.

3. Procédé selon l'une des revendications précédentes, **caractérisé en ce que** l'étape de détection de la position du point lumineux (10) sur la surface d'étalonnage (310) est répartie sur deux unités de détection (112, 114) du dispositif de détection (110), une première unité de détection (112) détectant le point lumineux (10) sur la surface d'étalonnage (310) et une deuxième unité de détection (114) déterminant l'emplacement du point lumineux (10) détecté sur la surface d'étalonnage (310).

4. Procédé selon l'une des revendications précédentes, **caractérisé en ce qu'**au moins un des critères suivants est utilisé comme critère d'interruption :
- consigne d'un nombre maximal de répétitions,
- consigne d'une distance (D) maximale entre la position détectée du point lumineux (10) sur la surface d'étalonnage (310) et le point d'étalonnage (20a, 20b, 20c).

5. Procédé selon l'une des revendications précédentes, **caractérisé en ce que** la modification de l'orientation du dispositif d'éclairage (120) a lieu selon un motif prédéfini.

6. Procédé selon la revendication 5, **caractérisé en ce que** le motif est une grille de recherche (30), en particulier une grille de recherche (30) 3x3x3 avec des distances (G) égales ou sensiblement égales des points de grille (32) individuels.

7. Procédé selon la revendication 6, **caractérisé en ce qu'**après avoir parcouru tous les points de grille (32) de la grille de recherche (30), le point de grille (32) associé à la position du point lumineux (10) qui présente la plus petite distance par rapport au point d'étalonnage (20a, 20b, 20c) est sélectionné comme point de départ pour la répétition avec la grille de recherche (30) suivante, en particulier avec une distance (G) réduite des points de grille (32).

8. Procédé selon l'une des revendications précédentes, **caractérisé en ce que** les étapes a) à i) sont exécutées au total pour au moins trois points d'étalonnage (20a, 20b, 20c) qui sont tous situés dans un plan dans lequel la surface d'étalonnage (310) s'étend.

9. Procédé selon l'une des revendications précédentes, **caractérisé en ce que** tous les points de correspondance définis sont mémorisés en tant que valeurs d'entrée pour une régulation suivante de l'orientation du dispositif d'éclairage (120) et/ou transmis à une unité de contrôle (130) destinée à effectuer la régulation de cette orientation.

10. Procédé selon l'une des revendications précédentes, **caractérisé en ce que** le point de correspondance trouvé pour le premier point d'étalonnage (20a) est utilisé pour exécuter les étapes pour ledit au moins un autre point d'étalonnage (20b, 20c).

11. Procédé selon l'une des revendications précédentes, **caractérisé en ce qu'**avant la production du point lumineux (10), un corps d'étalonnage (300) qui présente la surface d'étalonnage (310) est mis à disposition.

12. Système d'éclairage (100) avec un dispositif d'éclairage (120), un dispositif de détection (110) et une unité de contrôle (130) servant à réguler l'orientation du dispositif d'éclairage (120) afin de modifier la position de la zone d'éclairage produite par le dispositif d'éclairage (120) dans une direction d'éclairage (A), le dispositif de détection (110) et l'unité de contrôle (130) étant conçus pour la mise en oeuvre d'un procédé selon l'une des revendications 1 à 11.
